(19) **Europäisches Patentamt**
**European Patent Office**
**Office européen des brevets**

(11) **EP 1 774 903 B1**

(12) **EUROPEAN PATENT SPECIFICATION**

(45) Date of publication and mention
of the grant of the patent:
**11.06.2008 Bulletin 2008/24**

(51) Int Cl.:
*A61B 5/00* (2006.01)

(21) Application number: **05028513.9**

(22) Date of filing: **27.12.2005**

(54) **Metabolic rate measuring apparatus**

Gerät zur Messung der Stoffwechselaktivität

Dispositif de mesure du taux de métabolisme

(84) Designated Contracting States:
**DE FR GB**

(30) Priority: **14.10.2005 JP 2005300797**

(43) Date of publication of application:
**18.04.2007 Bulletin 2007/16**

(73) Proprietor: **HITACHI, LTD.**
**Chiyoda-ku**
**Tokyo 100-8280 (JP)**

(72) Inventors:
• **Uchida, Tsuyoshi, c/o Hitachi, Ltd.**
**Tokyo 100-8220 (JP)**

• **Mitsumaki, Hiroshi, c/o Hitachi, Ltd.**
**Tokyo 100-8220 (JP)**
• **Kiguchi, Masashi, c/o Hitachi, Ltd.**
**Tokyo 100-8220 (JP)**

(74) Representative: **Strehl Schübel-Hopf & Partner**
**Maximilianstrasse 54**
**80538 München (DE)**

(56) References cited:
**EP-A- 1 568 311     US-A1- 2003 130 567**
**US-A1- 2004 039 254**

**Description**

FIELD OF THE INVENTION

The present invention is directed to a metabolic rate measuring apparatus, which measures a plurality of physiological parameters without blood sampling, and then calculates a metabolic rate based on the plurality of physiological parameters.

BACKGROUND OF THE INVENTION

There have been several methods for measuring the metabolic rate. Indirect calorimetric methods measure the amount of oxygen consumed in order to produce the metabolism, instead of directly measuring the metabolic rate itself. Among indirect calorimetry there is an open circuit indirect calorimetric method, which measures the amount of consumed oxygen and carbon dioxide, by putting a mask on the subject face and by collecting the breathed air. This method is used for measuring the variation of metabolic rate in course of physical exercise and during rest. As wearing a mask for a long period of time is difficult, this method is applied for the metabolic rate measurement of a short period of time.

For measuring the metabolic rate for a longer time, a closed circuit indirect calorimetric metabolic rate measurement method is used, which put the subject in a small sealed chamber to measure the consumption rate of oxygen and production rate of carbon dioxide therein. This method is applicable to the metabolic rate measurement during rest and sleep, which method is fewer burdens to the subject, suitable for the measurement of longer period. There is also another method in which the subject drinks water including stable isotopes of hydrogen and oxygen harmless to the human body, then the process to excreting the stable isotope in the urine is measured to measure the energy consumption from the intake to the excretion.

This measurement method measures the mean energy consumption rate in the quotidian life of the subject. Documents EP 1568311andJP-A No. 329542/2004 disclose a method and apparatus for calculating the blood sugar value by means of the relationship between the blood sugar level and parameters corresponding to a plurality of temperature measurements derived from the body surface and the blood oxygen level.

SUMMARY OF THE INVENTION

So far the metabolic rate measurement requires a large apparatus, which is not easily applicable to daily personal use by an individual.

The present invention has its primary object to provide a method and apparatus, which facilitate and simplify the metabolic rate measurement by an individual at home.

Most calorimetric energy produced by the metabolism is consumed for the temperature homeostasis, and the redundant heat energy is released to the outside of human body. In other words this means that the calorimetric energy produced by the metabolism (thermogenesis) is equal to the sum of the calorimetric energy stored within the body (heat reserve) and the heat released to outside (radiation). This heat control mechanism of human body may be used for the calculation of metabolic rate based on the heat reserve and the heat radiation.

The metabolic rate measuring apparatus provided according to the present invention incorporates, an environmental thermometric sensor unit for measuring environmental temperature; a first position thermometric sensor unit for measuring temperature derived from a first position on body surface of a subject; a second position temperature acquiring mean acquiring temperature of a second position, the second position being different from said first position at the subject; a blood flow information acquiring mean acquiring information about the blood flow at said first position; a storage unit for storing the environment temperature, the temperature derived from said first position, the temperature of said second position, and relationship between metabolic rate and the blood flow; a processing unit for computing the metabolic rate by applying the environment temperature measured by said environment thermometric sensor unit, first position temperature measured by said first position thermometric sensor unit, second position temperature acquired by said second position temperature acquiring mean, and the blood flow information acquired by said blood flow information acquiring mean to the relationship stored in said storage unit; and a display unit for displaying the computational result from said processing unit.

The metabolic rate measurement apparatus according to the present invention further incorporates, an environment temperature sensor unit for measuring the environmental temperature; a body surface contact unit for contacting with a first position of the body surface, a cylinder member, contacted to said body surface contact unit and having an opening at one end; a radiation thermometric sensor mounted in the vicinity of the other end of said cylinder member, for measuring radiation heat from said first position; a blood flow information acquiring mean acquiring information about the blood flow at said first position; a light source for emitting light of at least two different wavelengths into the one end of said cylinder member; a light sensor unit for detecting light interacted with said body surface; a second position temperature acquiring mean acquiring temperature of a second position, the second position being different from said first position at said body surface; a processing unit for computing the metabolic rate by applying the environment temperature measured by said environment thermometric sensor unit, temperature of first position measured by said radiation thermometric sensor unit, temperature of second position acquired by said second position temperature acquiring mean, and blood flow rate information acquired by said blood flow information acquiring mean, and light detection result detected by said light detector unit to previously stored equation of the metabolic rate; and a display unit for displaying output from said processing unit.

The a second position temperature acquiring mean may be a thermometer so as to supply the output from the thermometer to the processing unit. The a second position temperature acquiring mean may also have an operation unit for inputting the body temperature as numerical data. The first position thermometric sensor unit may also be the one having a pad unit for placing a finger, and a temperature sensor for measuring the temperature derived from the first position of the finger placed on the pad unit.

According to the present invention, a simple metabolic rate measurement apparatus can be achieved.

BRIEF DESCRIPTION OF DRAWINGS

Fig. 1 shows a schematic block diagram indicative of the relationship between the measurements from various sensors and the parameter derived therefrom;
Fig. 2 shows a top plan view of metabolimeter according to the present invention;
Fig. 3 shows a top plan view of metabolimeter according to the present invention;
Fig. 4 shows a manual input operation of numerical values;
Fig. 5 shows the operation procedure of the apparatus;
Figs. 6A, 6B, and 6C show a detailed block diagram of a measuring unit;
Fig. 7 shows a schematic block diagram indicative of the flow of data processing within the apparatus according to the present invention;
Fig. 8 shows a schematic block diagram indicative of the location of data storage within the apparatus according to the present invention;
Fig. 9 shows a plot of the calculation value of metabolic rate according to the present invention vs. the metabolic rate measured by the Prior Art; and
Fig. 10 shows a plot of the calculated values of glucose concentration according to the present invention vs. measurements of glucose concentration by an enzymatic electrode method.

DETAILED DESCRIPTION OF THE PREFERRED EMBODIMENTS

Based on the principle of human body heat control mechanism, since the calorimetric energy produced by the metabolism (thermogenesis) is equal to the sum of the calorimetric energy stored within the body (heat reserve) and the heat released to outside (radiation), the following equation may be given:

$$(\textit{metabolic rate of whole body [thermogenesis]})$$
$$= (\textit{heat reserve of whole body}) + (\textit{heat radiation from whole body}) \quad \dots (1)$$

By defining T as the temperature in the body at the position $r_i$ of the body, $T_T$ as the tissue temperature at the body position $r_i$, and $\alpha l$ as the heat capacity at the body position ri, the heat reserve of the whole body may be the sum of the reserve at each part of the body, and can be given by the following equation:

$$\textit{Heat reserve of the whole body} = \sum_i \alpha_i \{T(\mathbf{r}_i) - T_T(\mathbf{r}_i)\} \quad \dots(2)$$

Equation 2 designates the amount of heat at each part of the body, and here the arterial blood temperature at a body depth is used as the effective value representative of the internal body temperature, and the temperature Tc as the effective value representative of the body tissue temperature. The heat capacity can be represented by $\alpha$ as the

effective value representative of the heat capacity of the whole body. The term $\alpha$ may be a value depending on the composition, density, and body weight of human body of each individual. From the foregoing the heat reserve of the whole body can be given by the following equation:

$$Heat\ reserve\ of\ whole\ body = \alpha(Ta - Tc) \qquad (3)$$

The heat radiation in general is through the conduction, radiation, convection, and evaporation. The conduction and radiation may have small amount of calorimetric heat when an individual is wearing clothes and can be neglected. The evaporation may also be neglected, provided that the temperature is controlled without perspiration in a room. When considering therefore the heat radiation by the convection between the body surface and the surrounding air, The heat radiation of the whole body may be the sum of the heat radiation from the body surface, and by defining the skin temperature at the body surface point $r_j$ as $T_S$, external temperature in contact with the body surface point $r_j$ as $T_{OUT}$, convection heat conduction rate at the body surface point $r_j$ as $\beta_j$, the following equation can be given:

$$Heat\ radiation\ of\ the\ whole\ body = \sum_j \beta_j \left\{ T_S(\mathbf{r}_j) - T_{OUT}(\mathbf{r}_j) \right\} \quad \cdots(4)$$

Equation 4 considers the heat amount at each part of body, where defining the skin temperature of the finger $T_{FS}$ as the effective value representative of the body surface temperature, the room temperature $T_R$ as the effective value representative of the external temperature. For the convection heat conduction rate, $\beta$ is defined as the effective value representative of the convection heat conduction rate of the entire surface area of the whole body. This $\beta$ is a value depending on the surface area and worn clothes of each individual. The heat radiation from the whole body may be given by the following equation:

$$Heat\ radiation\ from\ whole\ body = \beta(T_{FS} - T_R) \qquad (5)$$

In this preferred embodiment, the reason why a finger is used for a part representative of the heat radiation from the whole body is that the finger is sensitive to the change in the ambient temperature, expresses sufficiently largely the difference of skin temperature and radiation, as well as has a small local heat production due to less muscle to reflect the change in the whole body.

The metabolic rate of whole body may be given from the equations (1), (3), and (5) as following equation:

$$Metabolic\ rate\ of\ whole\ body\ (thermogenesis)$$
$$= \alpha(Ta - Tc) + \beta(T_{FS} - T_R) \qquad \cdots(6)$$

From the conservation law of heat flow in the finger, the incoming heat to the finger and the outgoing heat from the finger can be thought to be in equilibrium. For the finger tissue, Now defining the arterial temperature in depth of the finger as $T_{Fa}$, the heat conduction rate of the finger tissue as 1, blood flow rate in the finger tissue as $\omega_b$, the specific heat of blood as $c_b$, convection heat conduction rate of the finger surface as $\kappa$, the conservation law of heat flow in the finger can be given by the following equation, where $\chi$ designates to a factor of proportionality.

$$\lambda(\chi \omega_b c_b T_{Fa} - T_{FS}) = \kappa(T_{FS} - T_R) \qquad \cdots(7)$$

The left part indicates the heat displacement from the artery to the tissue at the finger surface, which is also based on the living tissue heat displacement equation by Penn (Pennes H. H., "Analysis of tissue and arterial blood temperatures in the resting human forearm" J. applied physiology, 1, 93-122(1948)).

Here assuming that the artery temperature in depth of the finger TFa is in relation to the arterial blood temperature Ta, and $T_{Fa} = \sigma Ta + \tau$, then the equation (7) may be written as follows:

$$T_a = \frac{1}{\sigma \chi c_b} \left\{ \frac{\kappa}{\lambda} (T_{FS} - T_R)/\omega_b + T_{FS}/\omega_b \right\} - \frac{\tau}{\sigma} \quad \cdots (8)$$

By substituting equation (8) with equation (6), equation (9) can be yielded. (Metabolic rate of whole body [thermogenesis])

$$= \frac{\alpha}{\sigma \chi c_b} \left\{ \frac{\kappa}{\lambda} (T_{FS} - T_R)/\omega_b + T_{FS}/\omega_b \right\} + \beta(T_{FS} - T_R) - \alpha T_c - \frac{\alpha \tau}{\sigma}$$

$$= a(T_{FS} - T_R)/\omega_b + bT_{FS}/\omega_b + c(T_{FS} - T_R) + dT_c + e \quad \cdots (9)$$

$$a = \frac{\alpha}{\sigma \chi c_b} \frac{\kappa}{\lambda}, \quad b = \frac{\alpha}{\sigma \chi c_b}, \quad c = \beta, \quad d = -\alpha, \quad e = -\frac{\alpha \tau}{\sigma}$$

In order to user equation (9), the relational factors a to e must be determined, which differ from person to person. To do this, the metabolic rate of an individual is measured to conduct a multiple regression analysis of the factor of each individual based on equation (9) and the metabolic rate measurements to derive the metabolic rate.

According to the modeled equations as above, The metabolic rate can be determined by measuring the temperature of finger surface $T_{FS}$ as the effective value representative of the body surface temperature, the room temperature $T_R$, The body temperature Tc as the effective value representative of the tissue temperature of the body, and the blood flow rate $\omega_b$ then by using the proportional factors measured separately. It should be noted here that although the finger is described as a typical measuring point in the body, any other part of the body surface is equally available. Some exemplary measurement points of the body tissue temperature may be selected from such as the sublingual, infra-axillary, or rectum temperature, such that the temperature is measured on the points different from the body temperature measurement positions as effective values representative of the body surface temperature, in order to obtain the temperature corresponding to the body tissue temperature. The temperature measurement locations used as effective value representative of the body surface temperature may also include, for example, one of the limbs instead of the finger surface.

Since the measurement of metabolic rate requires a massive apparatus, the metabolic rate may be determined which corresponds to the blood glucose concentration by means of the relationship between the blood glucose concentration that is the source of energy, and the metabolic rate. The relationship between the metabolic rate of whole body and the glucose concentration may be given by the following equation, by defining the calorimetric value produced by the glucose calorigenic production at the body part rk as QG(rj), and the calorimetric value produced by the heat production from any substance other than the glucose such as the lipids and aminoic acids as QΠ(rj), thus the metabolic rate of the whole body is the calorimetric sum produced on each body part:

*(Metabolic rate of whole body [thermogenesis])*

$$= \sum_k \left\{ QG(\mathbf{r}_k) + Q\Pi(\mathbf{r}_k) \right\} \quad \cdots (10)$$

Equation (10) considers the calorimetric value of each body part, however, when assuming that the mean value of intracellular glucose concentration Gc of the whole body is in proportion to the metabolic rate by the intracellular glucose, the mean intracellular concentration II of a substance other than the glucose in the whole body is in proportion to the metabolic rate by the substrate other than the intracellular glucose, and the factors of proportion is designated as A and B, the metabolic rate of the whole body may be described by the following equation:

*Metabolic rate of whole body = AGc + BΠ   ...(11)*

From equations (9) and (11), the following equation can be derived:

$$AG_c = a(T_{FS} - T_R)/\omega_b + bT_{FS}/\omega_b + c(T_{FS} - T_R) + dT_c + e - B\Pi \quad \cdots(12)$$

According to the foregoing modeled equations, by measuring the finger surface temperature $T_{FS}$ as the effective value representative of body surface temperature, the room temperature $T_R$, the body temperature Tc as the effective value representative of the body tissue temperature, and the blood flow rate $\omega_b$ and then by applying the proportional factors measured separately, the metabolic rate AGc corresponding to the blood glucose can be measured.

To improve the precision, consider the tissue oxygen saturation. The heat radiation of the whole body is in proportion to the amount of used oxygen, thus a proportional expression Gc + $\Pi \propto$ [$O_2$ consumption]. Tissue oxygen saturation $StO_2$ means the $O_2$ consumption, therefore can be given by the following expression:

$$StO_2 = a'\, Gc + b'\, \Pi \quad \ldots (13)$$

From the equations (12) and (13), the following equation (14) can be yielded.

$$\left(\frac{Ab' - a'B}{b'}\right)G_c = a(T_{FS} - T_R)/\omega_b + bT_{FS}/\omega_b + c(T_{FS} - T_R) + dT_c + e - \frac{B}{b'}StO_2 \quad \cdots(14)$$

According to the foregoing modeled equation, by measuring the finger surface temperature $T_{FS}$ as the effective value representative of body surface temperature, the room temperature $T_R$, the body temperature Tc as the effective value representative of the body tissue temperature, and the blood flow rate $\omega_b$, and the tissue oxygen saturation $StO_2$, then by applying the proportional factors measured separately, ((ab' -a'b)/b')Gc which is the metabolic rate corresponding to the blood glucose, can be measured.

Equation (15) can be yielded by rewriting the factors in equation (14), to calculate the glucose concentration.

$$G_c = a(T_{FS} - T_R)/\omega_b + bT_{FS}/\omega_b + c(T_{FS} - T_R) + dT_c + eStO_2 + f \quad \cdots(15)$$

$$a = \left(\frac{b'}{ab' - a'b}\right)\frac{\alpha}{\sigma\chi c_b}\frac{\kappa}{\lambda}, \quad b = \left(\frac{b'}{ab' - a'b}\right)\frac{\alpha}{\sigma\chi c_b}$$

$$c = \left(\frac{b'}{ab' - a'b}\right)\beta, \quad d = -\left(\frac{b'}{ab' - a'b}\right)\alpha$$

$$e = -\left(\frac{b'}{ab' - a'b}\right)\frac{b}{b'}, \quad f = -\left(\frac{b'}{ab' - a'b}\right)\frac{\alpha\tau}{\sigma}$$

Now the preferred embodiment of the present invention will be described in greater details with reference to the accompanying drawings herein below.

First, the finger surface temperature and the room temperature may be readily measured if the thermometric resistance or the radiation thermometer for measuring the radiation heat is used for example. The body temperature may be measured with an electronic thermometer equipped with the thermometric resistor or an auditory meatus thermometer using the radiation thermometer may be relatively easily used to measure the sublingual, axilla, rectum, or tympanic membrane temperature. The blood flow rate may be measured with a laser blood flow meter using for example the Doppler effect. The body temperature can also be measured by adding a minute amount of heat to a finger to evaluate the temperature change. The tissue oxygen saturation can be measured uninvasively with a pulse oxymeter or tissue oxygen saturation meter equipped with a feature for determining optically oxygen saturation.

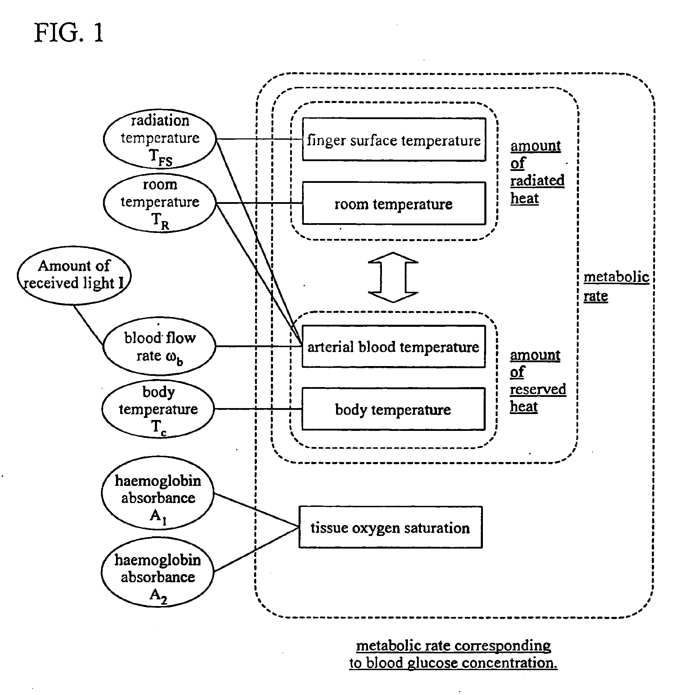

Now referring to Fig. 1, which shows a schematic block diagram illustrating the relationship between the measurements of various sensors and the parameters derived therefrom. Now the finger surface temperature $T_{FS}$ is measured using a radiation thermometer and the room temperature $T_R$ is measured with a thermistor. A thermometer connected to the apparatus or one commercially available is used for the measurement of body temperature Tc. The blood flow rate $\omega_b$ is calculated by measuring the photointensity I at the wavelength in relation to the haemoglobin absorbance. Parameters associated with the arterial blood temperature are derived from the finger body temperature TFS and the blood flow rate $\omega_b$. Parameters associated with the haemoglobin oxygen saturation are derived from the measurement using absorbance $A_1$ and $A_2$ of at last two different wavelengths in relation to the haemoglobin absorbance.

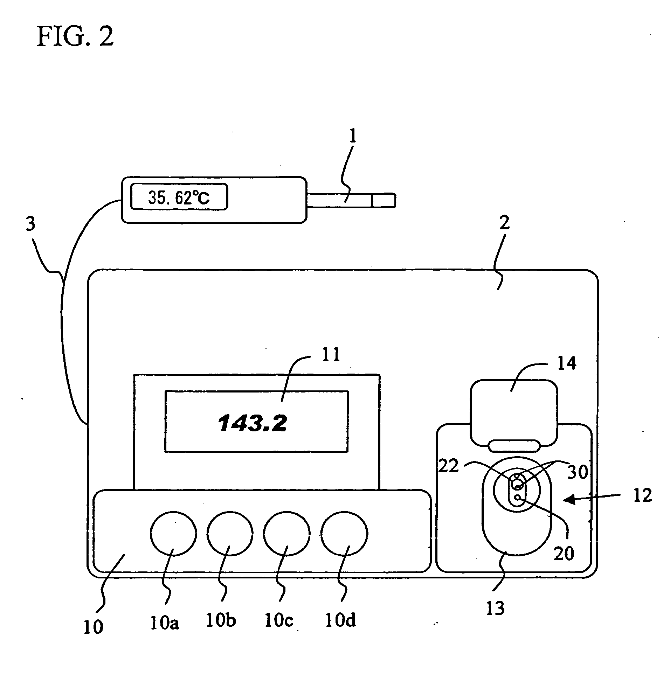

Now referring to Fig. 2, which is a top plan view of a metabolimeter according to the present invention. This apparatus uses the finger cushion skin as the body surface, however any other part of the body may be used instead, according to the measurement principle.

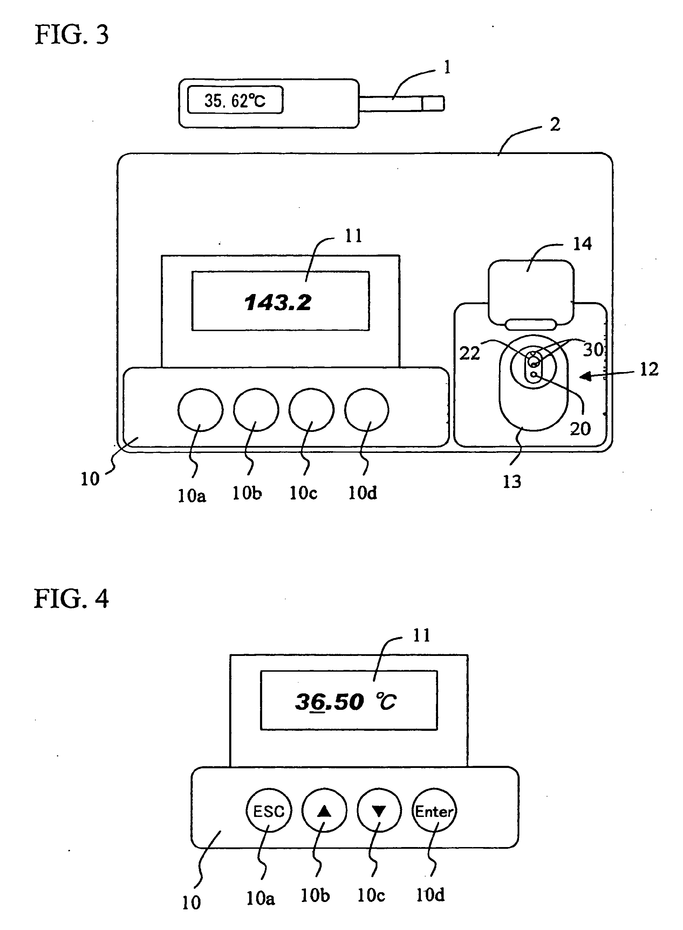

The apparatus is composed of a body temperature measuring unit 1 for measuring body temperature, and a main body 2 for measuring the room temperature, finger surface temperature, blood flow rate, and tissue oxygen saturation. The body temperature measuring unit 1 and the main body 2 are connected with a wiring 3. In the present embodiment, the body temperature measuring unit 1 is connected to the main body 2, and the body temperature data measured by the body temperature measuring unit 1 is transferred to the main body 2. However using a commercially available thermometer in common may be used to input the body temperature data via the operation unit 10 of the main body 2. Fig. 3 shows a top plan view of the metabolimeter according to the present invention with the body temperature and blood flow being measured with a generic device.

The body temperature data can be input by way of an example through a display screen as shown in Fig. 4. In this example 36.50 %%DEGREE is displayed as the reference, the ones place is underscored to indicate that this digit is to be input. In this state buttons 10b and 10c are used to change the number. More specifically, button 10b is pushed to increase the displayed value while the button 10c is pushed to decrease the value so as for the input to be equal to the measured body temperature, then the button 10d is used to enter the ones place. Thereafter the numerical input is continued in a similar manner for the one and two decimal places to complete the body temperature data input. When the button 10d is pushed by error, button 10a is pushed to undo the input.

On the top of the apparatus an operation unit 10, a measuring unit 12 on which a finger subject to be measured is placed, a display unit 11 for displaying the measurement result, apparatus status and the measurements. Four push buttons 10a to 10d are arranged in the operation unit 10 for operating the apparatus. A cover 14 is attached on the measuring unit 12, a finger pad unit 13, having an ellipsoid circumference is under the cover 14 (the cover is opened in the figure). In the finger pad unit 13 an open end 22 of the radiation thermometric sensor unit, optical sensor unit 30, and temperature sensor unit 20 are provided.

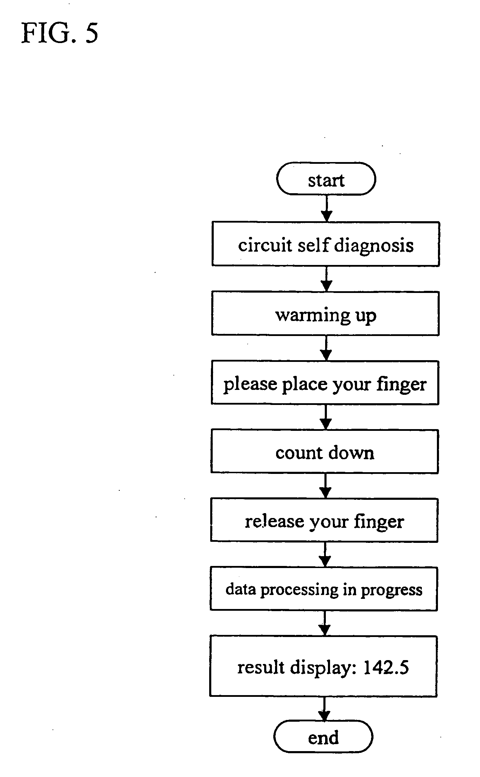

Now referring to Fig. 5, which shows the operation procedure of the apparatus. By pushing the button on the operation unit 10 to power on the apparatus, "warming up" is displayed on the display unit 11 and the electronics in the apparatus is warmed up. At the same time a checking program is loaded to automatically check the electronics. When "warming up" sign is over, an instruction "please place your finger" is displayed on the display unit 11. Once a finger is placed on the finger pad unit 13, the display unit 11 displays a down count. When the count down is complete, the display unit 11 indicates another instruction "release your finger". Once the finger is released from the finger pad unit 13, the display unit 11 displays a message "data processing in progress". Shortly after, the display unit 11 displays a metabolic rate corresponding to the blood glucose concentration. At this point, the displayed glucose level is stored in an IC card along with the date and time. After reading out the metabolic rate displayed, pushing the button on the operation unit causes the apparatus to cycle, and a message "please place your finger" on the display unit 11 for waiting next measurement appears in one minute.

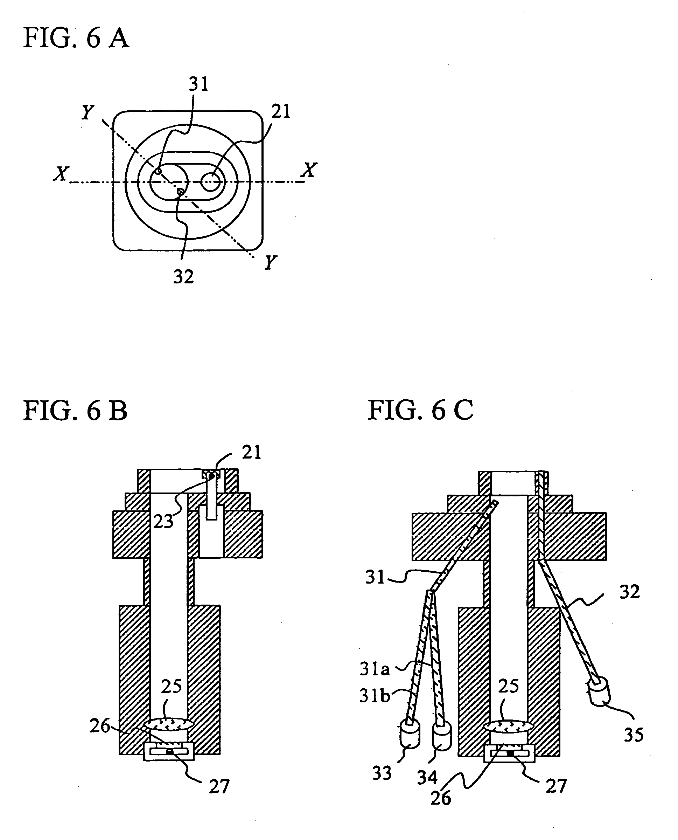

Now referring to Figs. 6A to 6C, which shows the details of the measurement unit; Fig. 6A is a top plan view, Fig. 6B is a cross-sectional view taken along with the line X-X, and Fig. 6C is another cross-sectional view taken along with the line Y-Y.

Now the temperature measurement with the metabolimeter according to the present invention will be described. An infrared lens 25 is located in a position within the apparatus to see through the subject (finger cushion) placed on the finger pad unit 13. Beneath the infrared lens 25 a pyroelectric detector 27 is placed with an infrared transmissible window 26 therebetween. A thermistor 23 is used for measuring the room temperature, to define the temperature immediately prior to placing a finger as the room temperature.

The temperature sensor unit of the preferred embodiment has three temperature sensors, for measuring three different temperature values as follows-(1) radiation temperature of the finger (pyroelectric detector 27): $T_{FS}$ (2) room temperature (thermistor 23): $T_R$ and (3) body temperature (body temperature measuring unit 1): Tc

Next the optical sensor unit 30 will be described in greater details. The optical sensor unit measures the haemoglobin concentration and the haemoglobin oxygen saturation needed for determining the tissue oxygen saturation. The measurement of the haemoglobin concentration and haemoglobin oxygen saturation requires the optical density measurement with at least two different wavelengths. Fig. 6C shows an exemplary arrangement for two wavelength measurements by means of two light sources 33 and 34, and one single detector 35.

The optical sensor unit 30 houses the ends of two optical fibers 31 and 32. The optical fiber 31 is an optical fiber for light emission, and the optical fiber 32 is for receiving lights. As shown in Fig. 6C, the optical fiber 31 is connected to two optical fiber branches 31a and 31b, at the end of which two light emission diodes (LED) for two different wavelengths are mounted. At the end of the photoreception optical fiber 32 a photodiode 35 is provided. The LED 33 emits the light of wavelength 830 nm, while the LED 34 emits the light of wavelength 780 nm. These two near-infrared lights serve for the measurement of concentration variation of oxygenated and reduced haemoglobin as well as the measurement of total haemoglobin concentration variation, which corresponds to the sum of two state haemoglobin and to the volume of blood.

Two LEDs 33 and 34 emit light in a time-sharing manner, and the light beam emitted from those LEDs 33 and 34 is transmitted through the light emission optical fiber 31 to the finger of the subject. The light emitted to the finger is diffused and reflected by the finger skin and then is incident to photoreception optic fiber 32 to be detected by the detector 35. At the time when the light emitted to the finger is diffused and reflected by the finger skin, the light passes through the skin and penetrates into the tissue so that it is absorbed by the haemoglobin in the blood stream flowing through the capillaries. The measurement data from the detector 35 indicates the reflectance R, from which the absorbance may be approximately calculated as log (1 / R). By emitting light of wavelength 830 nm and that of 780 nm, measuring the reflectance R for both wavelengths, as well as calculating log (1 / R), the absorbance A1 for wavelength 830 nm and the absorbance A2 for wavelength 780 nm are both determined.

When defining the reduced haemoglobin concentration as [Hb], oxygenated haemoglobin concentration as [HbO$_2$], the absorbance A$_1$ and A$_2$ may be given by the following equation:

$$A_1 = a \times ([Hb] \times A_{Hb}(830nm) + [HbO_2] \times A_{HbO_2}(830nm))$$

$$= a \times ([Hb] + [HbO_2]) \times A_{HbO_2}(830nm)$$

$$A_2 = a \times ([Hb] \times A_{Hb}(780nm) + [HbO_2] \times A_{HbO_2}(780nm))$$

$$= a \times ([Hb] + [HbO_2]) \times ((1 - \frac{[HbO_2]}{[Hb]+[HbO_2]}) \times A_{Hb}(780nm) + \frac{[HbO_2]}{[Hb]+[HbO_2]} \times A_{HbO_2}(780nm))$$

$$\cdots(16)$$

A$_{Hb}$(830 nm) and A$_{Hb}$(780 nm), A$_{HbO2}$(830 nm) and A$_{HbO2}$(780 nm) are molar absorbance coefficient of reduced haemoglobin, and oxygenated haemoglobin, respectively, and are known in the art. a indicates a proportional index. Haemoglobin concentration [Hb] + [HbO$_2$], Haemoglobin oxygen saturation [HbO$_2$]/([Hb] + [HbO$_2$]) may be given from the equation above as follows:

$$[Hb]+[HbO_2] = \frac{A_1}{a \times A_{HbO_2}(830nm)}$$

$$\frac{[HbO_2]}{[Hb]+[HbO_2]} = \frac{A_2 \times A_{HbO_2}(830nm) - A_1 \times A_{Hb}(780nm))}{A_1 \times (A_{HbO_2}(780nm) - A_{Hb}(780nm))} \quad \cdots(17)$$

It should be understood by those skilled in the art that although in this embodiment the absorbance measurement by using two wavelengths for determining the haemoglobin concentration and haemoglobin oxygen saturation has been described, absorbance measurement by using three or more wavelengths is also applicable, and may decreases the affection of interference to increase thereby the measurement precision.

The blood flow rate may be given from the frequency spectrum of the 780 nm light originated from the light emission diode 34 and diffused and reflected by the finger, as follows:

$$Tissue\ blood\ flow\ rate = k_1 \int \omega P(\omega) d\omega / I^2 \quad \cdots(18)$$

Where k$_1$ designates to the proportional index, $\omega$ to the angular frequency, P($\omega$) to the signal power spectrum, I to the amount of light received.

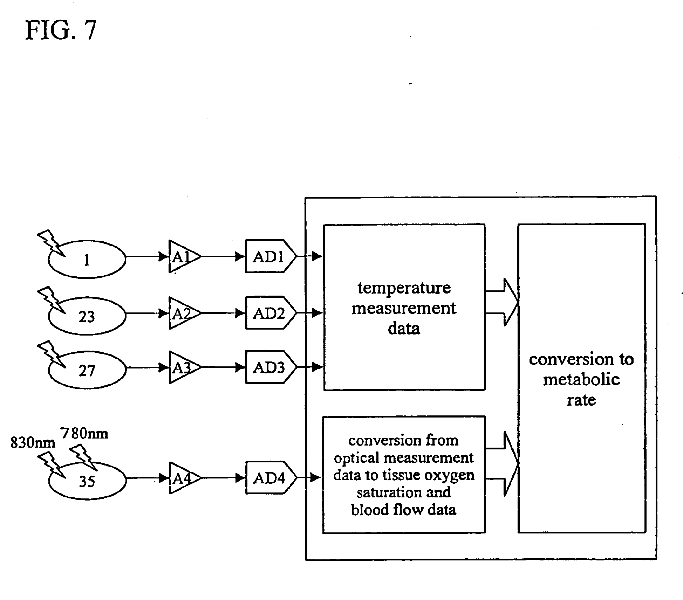

Now referring to Fig. 7, which shows diagrammatically a schematic block diagram illustrating the flow of data processing in the apparatus. The apparatus according to the preferred embodiment equips four sensor units each of which incorporates the body temperature measuring unit 1, a thermistor 23, a pyroelectric detector 27, and a photodiode 35. The photodiode 35 measures the absorbance of wavelength 830 nm and that of wavelength 780 nm. Five measurements are therefore input into the apparatus.

These five analog signals pass through respective amplifiers A1 to A4, then digitized by the A/D converters AD1 to AD4. From digitized values five physiological parameters (body temperature, room temperature, finger surface temperature, blood flow rate, tissue oxygen saturation) are calculated.

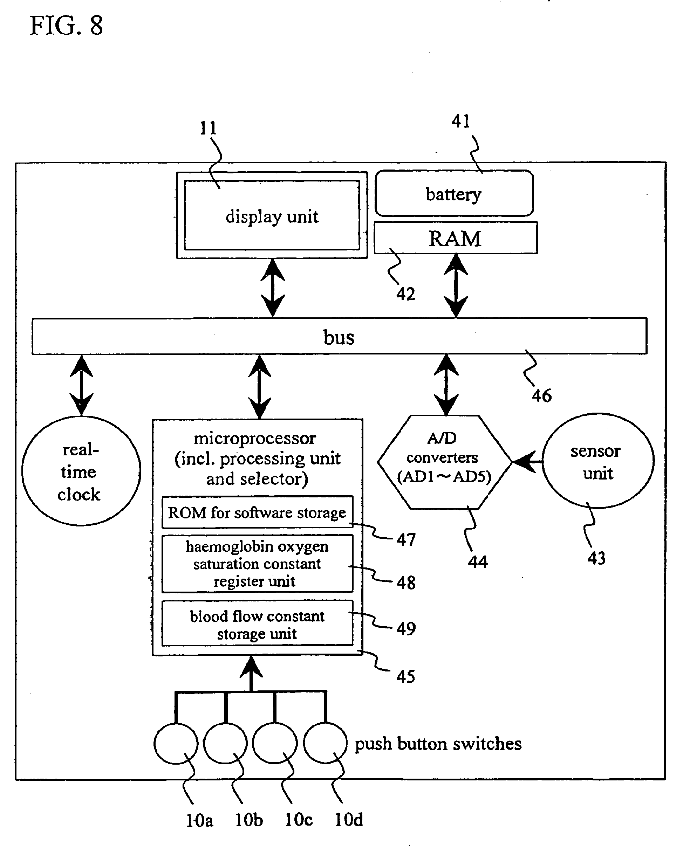

The conversion calculation is conducted from these five physiological parameters to the metabolic rate to be ultimately displayed. Now referring to Fig. 8, which shows the functional block diagram of the apparatus. The apparatus is driven by a battery 41. The signals measured by a sensor unit 43 including a temperature sensor and an optical sensor are fed to A/D converters 44 each arranged in correspondence with the respective signal (A/D converter AD 1 to AD4) to be converted to digital signals. There are peripherals of a microprocessor 45, including A/D converters AD1 to AD4, LCD display unit 11, RAM 42, which are accessed by the microprocessor 45 via a bus 46, respectively. Push buttons 10a to 10d are each connected to the microprocessor 45. The microprocessor 45 receives external instructions by pushing the buttons 10a to 10d.

A ROM 47 incorporated in the microprocessor 45 stores any programs necessary for the processing. The microprocessor 45 also includes a haemoglobin oxygen saturation constant register unit 48 for storing the constant for the haemoglobin oxygen saturation, and a blood flow rate constant register unit 29 for storing the constant for the blood flow rate. The calculation program, after having performed the measurement of the finger, calls the optimum constants from the $Hb/O_2$ constant register unit and the blood flow constant register unit to start calculation. The memory area required for the processing is allocated in the RAM 42, which is also incorporated in the apparatus. The calculation result will be displayed on the display unit 11.

In addition, although the apparatus calculates the metabolic rate therein in the present embodiment, the physiological parameters may also be stored in an IC card, and another PC reads the data on the IC card to calculate the metabolic rate.

The ROM stores any program components necessary for the processing, especially function for determining the metabolic rate. The function is defined as follows. First, the metabolic rate is expressed as equation (9). The indexes a to e in equation (9) are predetermined based on a plurality of measurements. The procedure for determining the a to e ($a_i$, (i=0, 1, 2, 3, 4)) is as follows.

(1) prepare physiological parameters and multiple regression indicative of the metabolic rate of another measurement.
(2) determine a normal equation (simultaneous equation) with respect to the physiological parameters from the equation obtained by the least square method.
(3) determine the indexes $a_i$ (i=0, 1, 2, 3, 4) from the normal equation and assign the value into the multiple regression.

Glucose concentration is expressed as equation (15). The indexes a to f in equation (15) are predetermined based on a plurality of measurements. The procedure to determine a to f ($a_i$, (i = 0, 1, 2, 3, 4, 5) may be the same as that of the metabolic rate. Now an exemplary case of glucose concentration is shown here. First, the following regression is created, which indicates the relationship between the blood glucose concentration Gc and physiological parameters $X_1 = (T_{FS} - T_R)/\omega_b$, $X_2 = T_{FS}/\omega_b$, $X_3 = (T_{FS} - T_R)$, $X_4 = T_C$, $X_5 = StO_2$.

$$C = f(X_1, X_2, X_3, X_4, X_5)$$
$$= a_0 + a_1 X_1 + a_2 X_2 + a_3 X_3 + a_4 X_4 + a_5 X_5 \qquad \cdots (19)$$

Next, the least square method is used to define a multiple regression so as to minimize the error with the blood glucose concentration measurements Gc measured by an enzymatic electrode method. By defining the sum of squares of the residual error as D, D may be given by the following equation:

$$D = \sum_{i=1}^{n} d_i^2$$

$$= \sum_{i=1}^{n} (C_i - f(X_{i1}, X_{i2}, X_{i3}, X_{i4}, X_{i5}))^2$$

$$= \sum_{i=1}^{n} \{C_i - (a_0 + a_1 X_{i1} + a_2 X_{i2} + a_3 X_{i3} + a_4 X_{i4} + a_5 X_{i5})\}^2 \quad \cdots(20)$$

Since where the sum of squares of residual becomes minimum is when equation (20) partially differentiated by $a_0$, $a_1$, ..., $a_5$ becomes 0, the following equation can be given:

$$\frac{\partial D}{\partial a_0} = -2 \sum_{i=1}^{n} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_1} = -2 \sum_{i=1}^{n} X_{i1} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_2} = -2 \sum_{i=1}^{n} X_{i2} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_3} = -2 \sum_{i=1}^{n} X_{i3} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_4} = -2 \sum_{i=1}^{n} X_{i4} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_5} = -2 \sum_{i=1}^{n} X_{i5} \{C_i - (a_0 + a_1 X_{i1} + a_2 X_{i2} + a_3 X_{i3} + a_4 X_{i4} + a_5 X_{i5})\} = 0 \quad \cdots(21)$$

Now defining mean of C, $X_1$ to $X_5$ as $C_{mean}$, $X_{1mean}$ to $X_{5mean}$, then $X_{imean} = 0$ (i = 1 - 5), the following equation (22) may be given from equation (19):

$$a_0 = C_{mean} - a_1 X_{1mean} - a_2 X_{2mean} - a_3 X_{3mean} - a_4 X_{4mean} - a_5 X_{5mean}$$

$$= C_{mean} \quad \cdots(22)$$

Variation and covariation between normalized parameters are expressed by equation (23), and covariation between normalized parameters $X_i$ (i = 1 - 5) and C are expressed by equation (24):

$$S_{ij} = \sum_{k=1}^{n} (X_{ki} - X_{imean})(X_{kj} - X_{jmean}) = \sum_{k=1}^{n} X_{ki} X_{kj} \quad (i, j = 1, 2, ..5) \quad \cdots(23)$$

$$S_{iC} = \sum_{k=1}^{n} (X_{ki} - X_{imean})(C_k - C_{mean}) = \sum_{k=1}^{n} X_{ki}(C_k - C_{mean}) \quad (i = 1, 2, ..5) \quad \cdots(24)$$

Now assigning equations (22), (23), and (24) into equation (21) to digest to yield a simultaneous equation (normal equation) (25). By solving equation (25) $a_1$ - $a_5$ may be given.

$$a_1 S_{11} + a_2 S_{12} + a_3 S_{13} + a_4 S_{14} + a_5 S_{15} = S_{1C}$$
$$a_1 S_{21} + a_2 S_{22} + a_3 S_{23} + a_4 S_{24} + a_5 S_{25} = S_{2C}$$
$$a_1 S_{31} + a_2 S_{32} + a_3 S_{33} + a_4 S_{34} + a_5 S_{35} = S_{3C}$$
$$a_1 S_{41} + a_2 S_{42} + a_3 S_{43} + a_4 S_{44} + a_5 S_{45} = S_{4C}$$
$$a_1 S_{51} + a_2 S_{52} + a_3 S_{53} + a_4 S_{54} + a_5 S_{55} = S_{5C} \qquad \cdots (25)$$

Constant term $a_0$ may be given by equation (22). The $a_i$ (i = 0, 1, 2, 3, 4, 5) as defined as above is stored on the ROM as manufacturer supplied. In the actual measurement using the apparatus, normalized parameters $X_1$ to $X_5$ determined from the measurements are substituted for the regression equation (19) to calculate the glucose concentration. It should be noted here that the $a_i$ (i = 0, 1, 2, 3, 4, 5) may be defined for the user after delivery to store in the IC card, instead of as-manufactured basis.

A specific example of calculation of metabolic rate will be described in greater details herein below. The indexes of equation (9) are defined from the data of many measurements performed in advance on a number of persons, and the ROM of the microprocessor stores the following equation of metabolic rate.

$$Metabolic\ rate = 13415 - 308 T_c - 31(T_{FS} - T_R) + 38(T_{FS} - T_R)/\omega_b - 6.4 T_{FS}/\omega_b$$

As an example of measurements, the equation above is substituted with measurement data Tc = 36.45, $T_{FS}$ - $T_R$ = 7.80, ($T_{FS}$ - $T_R$)/$\omega_b$ = 13.11, $T_{FS}$/$\omega_b$ = 54.03 to yield 8.8 kJ. The metabolic rate measured by an indirect calorimetric measurement method in a closed circuit of the same is 8.7 kJ. The measurement data when the metabolic rate is obtained as 8.2 kJ in the indirect calorimetric measurement method in a closed circuit, i.e., Tc = 36.99, $T_{FS}$ - $T_R$ = 6.76, ($T_{FS}$ - $T_R$)/$\omega_b$ = 7.68, $T_{FS}$/$\omega_b$ = 35.41 may be substituted for the equation to obtain 7.9 kJ.

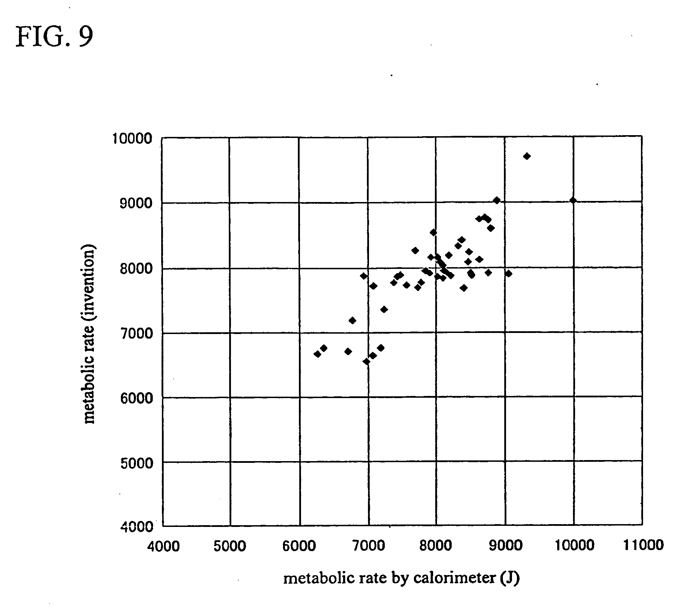

Now referring to Fig. 9, which shows a plot of measurements obtained from a plurality of subjects, with the ordinate plotting the calculated values of metabolic rate according to the present invention, and the abscissa plotting the measurements of metabolic rate by means of the indirect calorimetric measurement method in a closed circuit system. In the inventive method a better correlation is obtainable by calculating the metabolic rate from the measurement of human body temperature, finger temperature, and blood flow rate (correlative index = 0.82).

A specific example of calculation of glucose concentration will be described in greater details herein below. The index of equation (15) is defined from the data of many measurements performed in advance on a number of people, and the following calculation equation of glucose concentration is stored in the ROM of the microprocessor.

$$Gc = 2256.8 - 54.4 T_c + 8.7(T_{FS} - T_R) - 4.95(T_{FS} - T_R)/\omega_b - 0.7 T_{FS}/\omega_b - 182.3 StO_2$$

As a typical example of measurements, substituting measurement data Tc = 36.45, $T_{FS}$ - $T_R$ = 7.80, ($T_{FS}$ - $T_R$)/$\omega_b$ = 13.11, $T_{FS}$/$\omega_b$ = 54.03, $StO_2$ = 0.72 for the equation above may yield 148.1. The blood glucose concentration at that time is 178.6 mg/dl. Then substituting the measurement data at the time of blood glucose concentration 203.4 mg/dl, Tc = 36.52, $T_{FS}$ - $T_R$ = 7.81, ($T_{FS}$ - $T_R$)/$\omega_b$ = 11.30, $T_{FS}$/$\omega_b$ = 46.51, $StO_2$ = 0.49 for the above equation yields 201.5 mg/dl.

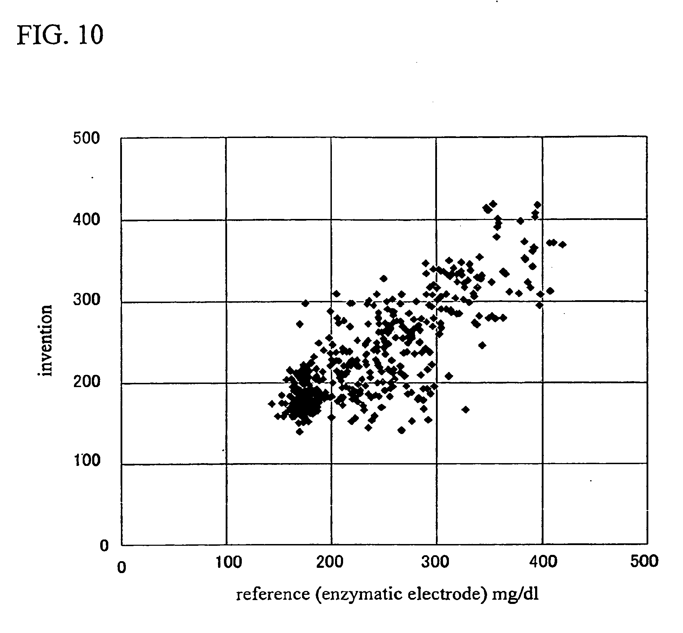

Now referring to Fig. 10, which shows a plot of measurements obtained from a plurality of subjects, with the ordinate plotting the calculated values of glucose concentration according to the present invention, and the abscissa plotting the measurements of glucose concentration by means of an enzymatic electrode method. In the inventive method a better correlation is obtainable by calculating the blood glucose concentration from the measurements of human body temperature, finger temperature, volume of supplied oxygen, and blood flow rate (correlative index = 0.84).

The metabolic rate can be calculated which corresponds to the blood glucose concentration by multiplying the calculated glucose concentration by the index A of equation (11).

It should be noted here that although the metabolic rate is investigated which correspond to the glucose concentration in the preferred embodiment, the metabolic rate may equally be based on the neutral fat (acylglycerol) and/or cholesterol as the metabolic rate of a substance other than the glucose, the equations may be defined from the blood concentration and measurements to determine the metabolic rate corresponding to the neutral fat and cholesterol concentration.

With respect to the calculated metabolic rate, or glucose concentration, or metabolic rate corresponding to the blood glucose concentration, the measurements accumulated from the group of healthy adults has the tendency of being different from those accumulated from the group of patients of such metabolic diseases as arteriosclerosis, heart diseases, glucose tolerance anomaly and the like. The calculated values may be clues as to the underlying diseases. The time-series display of accumulated metabolic rate allows facilitating the diagnosis of the tendency.

The present embodiment has the identical configuration as the preceding embodiment, the similar members are designated to the identical reference numbers and the detailed description of the parts already described in the preceding embodiment will be omitted.

The present invention may be embodied in other specific forms without departing from the spirit or essential characteristics thereof. For instance, (effect of the invention)

It is to be understood that the present invention is not to be limited to the details herein given but may be modified within the scope of the appended claims.

The foregoing description of the preferred embodiment of the invention has been presented for purposes of illustration and description. It is not intended to be exhaustive or to limit the invention to the precise form disclosed, and modifications and variations are possible in light of the above teachings or may be acquired from practice of the invention. The embodiment chosen and described in order to explain the principles of the invention and its practical application to enable one skilled in the art to utilize the invention in various embodiments and with various modifications as are suited to the particular use contemplated. It is intended that the scope of the invention be defined by the claims appended hereto, and their equivalents.

It is further understood by those skilled in the art that the foregoing description is a preferred embodiment of the disclosed device and that various changes and modifications may be made in the invention without departing from the scope thereof.

**Claims**

1. A metabolic rate measurement apparatus, comprising:

   an environmental thermometric sensor unit (23) for measuring the environmental temperature;
   a first position thermometric sensor unit (12; 27) for measuring a temperature derived from a first position on a body surface of a subject,
   second position temperature acquiring means (1) for acquiring a temperature of a second position, the second position being different from said first position at the subject;
   blood flow information acquiring means (30) for acquiring information about the blood flow at said first position;
   a storage unit for storing the environmental temperature, the temperature derived from said first position, the temperature of said second position, and a relationship between metabolic rate and the blood flow;
   a processing unit (45) for computing the metabolic rate by applying the environmental temperature measured by said environmental thermometric sensor unit (23), the first position temperature measured by said first position thermometric sensor unit (12; 27), the second position temperature acquired by said second position temperature acquiring means (1), and the blood flow information acquired by said blood flow information acquiring means (30) to the relationship stored in said storage unit; and
   a display unit (11) for displaying the computational result from said processing unit (45).

2. The apparatus of claim 1, wherein said second position temperature acquiring means (1) is comprised of a body thermometer, the output of said body thermometer being supplied to said processing unit (45).

3. The apparatus of claim 1, wherein said second position temperature acquiring means (1) has an operation unit for inputting the body temperature as numeric data.

4. The apparatus of claim 1, wherein said blood flow information acquiring means (30) has
   a light source (33, 34) for emitting light to said first position, and
   a photometer (35) for measuring the frequency spectrum of light diffused and reflected from said first position.

5. The apparatus of claim 1, wherein said blood flow information acquiring means (30) has an operation unit for inputting the blood flow rate as numeric data.

6. The apparatus of claim 1, further comprising oxygen saturation information acquiring means for acquiring information about the oxygen saturation at said first position, wherein
   said storage unit stores the temperature derived from said first position, the temperature of said second position, and the relationship between said blood flow rate and said oxygen saturation and the metabolic rate, and
said processing unit (45) calculates the metabolic rate by applying the environmental temperature measured by said environmental thermometric sensor unit (21), the first position temperature measured by said first position thermometric sensor unit (12; 27), the second position temperature measured by said second position temperature acquiring means (1), the blood flow information acquired by said blood flow information acquiring means (30), and the oxygen saturation acquired by said oxygen saturation information acquiring means to said relationship stored in said storage unit.

7. The apparatus of claim 6, wherein said oxygen saturation information acquiring means has
a light source (33, 34) for emitting light of at least two different wavelengths,
a detector (35) for detecting light originated from said light source (33, 34) and diffused by said body surface, and
means for calculating a haemoglobin oxygen saturation based on the detection results from said detector (35).

8. The apparatus of claim 6, wherein said oxygen saturation information acquiring means has an operation unit for entering the oxygen saturation as numerical data.

9. The apparatus of claim 1, wherein said first position thermometric sensor unit (12) has
a pad unit (13) for placing a finger, and
a temperature sensor (20) for measuring the temperature derived from said first position of the finger placed on said pad unit (13), said temperature sensor (20) preferably measuring a radiation temperature derived from said first position.

10. A metabolic rate measurement apparatus, comprising:

an environmental temperature sensor unit (23) for measuring the environmental temperature;
a body surface contact unit (13) for contacting with a first position of the body surface;
a cylinder member, contacted to said body surface contact unit (13) and having an opening at one end;
a radiation thermometric sensor (27) mounted in the vicinity of the other end of said cylinder member, for measuring radiation heat from said first position;
blood flow information acquiring means (30) for acquiring information about the blood flow at said first position;
a light source (33, 34) for emitting light of at least two different wavelengths into the one end of said cylinder member,
a light sensor unit (35) for detecting light interacted with said body surface;
second position temperature acquiring means (1) for acquiring a temperature of a second position, the second position being different from said first position at said body surface;
a processing unit (45) for computing the metabolic rate by applying the environmental temperature measured by said environmental thermometric sensor unit (23), the temperature of first position measured by said radiation thermometric sensor unit (27), the temperature of the second position acquired by said second position temperature acquiring means (1), the blood flow rate information acquired by said blood flow information acquiring means (30), and a light detection result detected by said light detector unit to a previously stored equation of the metabolic rate; and
a display unit (11) for displaying the output from said processing unit (45).

11. The apparatus of claim 10, wherein said second position temperature acquiring means (1) is
a body thermometer, or
an operation pad for entering the body temperature as numeric data.

12. The apparatus of claim 10, wherein
said first position is to be a part in one of four limbs of the subject to be measured, and
said radiation thermometric sensor (27) measures the radiation heat derived from said part.

13. The apparatus of claim 10, further comprising a data storage unit for storing output from said processing unit (45), wherein data stored in said data storage unit is displayed on said display unit (11) in a time-series manner.

14. The apparatus of claim 10, wherein said metabolic rate is
the metabolic rate of glucose, or
the metabolic rate of neutral fat and/or cholesterol.

**Patentansprüche**

1.  Gerät zur Messung der Stoffwechselaktivität, mit:

    einer Umgebungs-Thermometersensoreinheit (23) zum Messen der Umgebungstemperatur;
    einer thermometrischen Sensoreinheit erster Position (12; 27) zum Messen einer Temperatur, die von einer ersten Position auf einer Körperfläche eines Subjekts erlangt wird,
    einer Temperaturerfassungseinrichtung zweiter Position (1) zum Erfassen einer Temperatur einer zweiten Position, die sich von der ersten Position an dem Subjekt unterscheidet;
    einer Durchblutungsinformations-Erfassungseinrichtung (30) zum Erfassen von Information bezüglich der Durchblutung an der ersten Position;
    einer Speichereinheit zum Speichern der Umgebungstemperatur, der von der ersten Position erlangten Temperatur, der Temperatur der zweiten Position und einer Beziehung zwischen der Stoffwechselaktivität und der Durchblutung;
    einer Verarbeitungseinheit (45) zum Berechnen der Stoffwechselaktivität durch Anwenden der durch die Umgebungs-Thermometersensoreinheit (23) gemessenen Umgebungstemperatur, der durch die thermometrische Sensoreinheit erster Position (12; 27) gemessene Temperatur der ersten Position, der durch die Temperaturerfassungseinrichtung (1) erfasste Temperatur der zweiten Position, und der durch die Durchblutungsinformations-Erfassungseinrichtung (30) erfassten Durchblutungsinformation auf die in der Speichereinheit gespeicherte Beziehung; und
    einer Anzeigeeinheit (11) zum Anzeigen des Berechnungsergebnisses von der Verarbeitungseinheit (45).

2.  Gerät nach Anspruch 1, wobei die Temperaturerfassungseinrichtung zweiter Position (1) in einem Körperthermometer besteht, dessen Ausgabesignal der Verarbeitungseinheit (45) zugeführt wird.

3.  Gerät nach Anspruch 1, wobei die Temperaturerfassungseinrichtung zweiter Position (1) eine Betriebseinheit zum Eingeben der Körpertemperatur als numerische Daten aufweist.

4.  Gerät nach Anspruch 1, wobei die Durchblutungsinformations-Erfassungseinrichtung (30) aufweist:

    eine Lichtquelle (33, 34) zum Emittieren von Licht an die erste Position, und
    ein Photometer (35) zum Messen des Frequenzspektrums von Licht, das von der ersten Position gestreut und reflektiert wird.

5.  Gerät nach Anspruch 1, wobei die Durchblutungsinformations-Erfassungseinrichtung (30) eine Betriebseinheit zum Eingeben der Durchblutungsrate als numerische Daten aufweist.

6.  Gerät nach Anspruch 1, ferner mit einer Sauerstoffsättigungsinformations-Erfassungseinrichtung zum Erfassen von Information bezüglich der Sauerstoffsättigung an der ersten Position, wobei
    die Speichereinheit die von der ersten Position erhaltene Temperatur, die Temperatur der zweiten Position und die Beziehung zwischen der Durchblutungsrate und der Sauerstoffsättigung und der Stoffwechselaktivität speichert, und
    die Verarbeitungseinheit (45) die Stoffwechselaktivität berechnet, indem sie die durch die Umgebungs-Thermometersensoreinheit (21) gemessene Umgebungstemperatur, die durch die thermometrische Sensoreinheit erster Position (12; 27) gemessene Temperatur der ersten Position, die durch die Temperaturerfassungseinrichtung zweiter Position gemessene Temperatur der zweiten Position, die durch die Durchblutungsinformations-Erfassungseinrichtung (30) erfasste Durchblutungsinformation und die durch die Sauerstoffsättigungsinformations-Erfassungseinrichtung erfasste Sauerstoffsättigung auf die in der Speichereinheit gespeicherte Beziehung anwendet.

7.  Gerät nach Anspruch 6, wobei die Sauerstoffsättigungsinformations-Erfassungseinrichtung aufweist:

    eine Lichtquelle (33, 34) zum Emittieren von Licht wenigstens zweier verschiedener Wellenlängen,
    einen Detektor (35) zum Erfassen von aus der Lichtquelle (33, 34) emittiertem Licht, das durch die Körperfläche gestreut wurde, und
    eine Einrichtung zum Berechnen einer Hämoglobinsauerstoffsättigung auf Grundlage des Detektionsergebnisses von dem Detektor (35).

8.  Gerät nach Anspruch 6, wobei die Sauerstoffsättigungsinformations-Erfassungseinrichtung eine Betriebseinheit zum Eingeben der Sauerstoffsättigung als numerische Daten aufweist.

**9.** Gerät nach Anspruch 1, wobei die thermometrische Sensoreinheit erster Position (12) aufweist:

eine Auflageeinheit (13) zum Auflegen eines Fingers, und
einen Temperatursensor (20) zum Messen der von der ersten Position erlangten Temperatur des Fingers auf der Auflageeinheit (13), wobei der Temperatursensor (20) vorzugsweise eine von der ersten Position erhaltene Strahlungstemperatur misst.

**10.** Gerät zur Messung der Stoffwechselaktivität, mit:

einer Umgebungs-Temperatursensoreinheit (23) zum Messen der Umgebungstemperatur;
einer Körperflächenkontakteinheit (13) zum Kontaktieren einer ersten Position der Körperfläche;
einem Zylinderelement, das in Kontakt mit der Körperflächenkontakteinheit (13) steht und an einem Ende eine Öffnung aufweist;
einem Strahlungsthermometersensor (27), der in der Nähe des anderen Endes des Zylinderelements befestigt ist, zum Messen von Strahlungwärme von der ersten Position;
einer Durchblutungsinformations-Erfassungseinrichtung (30) zum Erfassen von Information bezüglich der Durchblutung an der ersten Position;
einer Lichtquelle (33, 34) zum Emittieren von Licht wenigstens zweier verschiedener Wellenlängen in das eine Ende des Zylinderelements;
einer Lichtsensoreinheit (35) zum Erfassen von Licht, das mit der Körperfläche wechselgewirkt hat;
einer Temperaturerfassungseinrichtung zweiter Position (1) zum Erfassen einer Temperatur einer zweiten Position, die sich von der ersten Position auf der Körperfläche unterscheidet;
einer Verarbeitungseinheit (45) zum Berechnen der Stoffwechselaktivität, indem die durch die Umgebungs-Thermometersensoreinheit (23) gemessene Umgebungstemperatur, die durch die Strahlungsthermometersensoreinheit (27) gemessene Temperatur der ersten Position, die durch die Temperaturerfassungseinrichtung zweiter Position (1) erfasste Position der zweiten Position, die durch die Durchblutungsinformations-Erfassungseinrichtung (30) erfasste Durchblutungsrateninformation, und ein durch die Lichtdetektoreinheit erfasstes Lichtdetektionsergebnis auf eine zuvor gespeicherte Gleichung der Stoffwechselaktivität angewendet wird; und
einer Anzeigeeinheit (11) zum Anzeigen des Ausgabesignals von der Verarbeitungseinheit (45).

**11.** Gerät nach Anspruch 10, wobei die Temperaturerfassungseinrichtung zweiter Position (1)
ein Körperthermometer, oder
ein Operationsfeld zum Eingeben der Körpertemperatur als numerische Daten ist.

**12.** Gerät nach Anspruch 10, wobei
die erste Position ein Teil eines der vier Glieder des zu vermessenden Subjekts ist, und
der Strahlungstemperatursensor (27) die von diesem Teil erlangte Strahlungswärme misst.

**13.** Gerät nach Anspruch 10, ferner mit einer Datenspeichereinheit zum Speichern des Ausgabesignals von der Verarbeitungseinheit (45), wobei in der Datenspeichereinheit gespeicherte Daten in zeitlicher Abfolge auf der Anzeigeneinheit (11) angezeigt werden.

**14.** Gerät nach Anspruch 10, wobei die Stoffwechselaktivität in
der Stoffwechselaktivität von Glucose oder
der Stoffwechselaktivität von neutralem Fett und/oder Cholesterin besteht.

**Revendications**

**1.** Dispositif de mesure de taux métabolique, comportant :

une unité de détection thermométrique environnementale (23) pour mesurer la température environnementale,
une unité de détection thermométrique de première position (12 ; 27) pour mesurer une température dérivée d'une première position sur une surface corporelle d'un sujet,
des moyens d'acquisition de température de seconde position (1) pour acquérir une température d'une seconde position, la seconde position étant différente de ladite première position sur le sujet,
des moyens d'acquisition d'informations de flux sanguin (30) pour acquérir des informations concernant le flux sanguin à ladite première position,

une unité de mémorisation pour mémoriser la température environnementale, la température dérivée de ladite première position, la température de ladite seconde position, et une relation entre un taux métabolique et le flux sanguin,

une unité de traitement (45) pour calculer le taux métabolique en appliquant la température environnementale mesurée par ladite unité de détection thermométrique environnementale (23), la température de première position mesurée par ladite unité de détection thermométrique de première position (12 ; 27), la température de seconde position acquise par lesdits moyens d'acquisition de température de seconde position (1), et les informations de flux sanguin acquises par lesdits moyens d'acquisition d'informations de flux sanguin (30), à la relation mémorisée dans ladite unité de mémorisation, et

une unité d'affichage (11) pour afficher le résultat de calcul de ladite unité de traitement (45).

2. Dispositif selon la revendication 1, dans lequel lesdits moyens d'acquisition de température de seconde position (1) sont constitués d'un thermomètre corporel, la sortie dudit thermomètre corporel étant délivrée à ladite unité de traitement (45).

3. Dispositif selon la revendication 1, dans lequel lesdits moyens d'acquisition de température de seconde position (1) ont une unité opérationnelle pour entrer la température corporelle sous forme de données numériques.

4. Dispositif selon la revendication 1, dans lequel lesdits moyens d'acquisition d'informations de flux sanguin (30) ont une source de lumière (33, 34) pour émettre une lumière vers ladite première position, et un photomètre (35) pour mesurer le spectre de fréquences d'une lumière diffusée et réfléchie par ladite première position.

5. Dispositif selon la revendication 1, dans lequel lesdits moyens d'acquisition d'informations de flux sanguin (30) ont une unité opérationnelle pour entrer le débit sanguin sous forme de données numériques.

6. Dispositif selon la revendication 1, comportant de plus des moyens d'acquisition d'informations de saturation en oxygène pour acquérir des informations concernant la saturation en oxygène à ladite première position, dans lequel ladite unité de mémorisation mémorise la température dérivée de ladite première position, la température de ladite seconde position, et la relation entre le débit sanguin et ladite saturation en oxygène et le taux métabolique, et ladite unité de traitement (45) calcule le taux métabolique en appliquant la température environnementale mesurée par ladite unité de détection thermométrique environnementale (21), la température de première position mesurée par ladite unité de détection thermométrique de première position (12 ; 27), la température de seconde position mesurée par lesdits moyens d'acquisition de température de seconde position (1), les informations de flux sanguin acquises par lesdits moyens d'acquisition d'informations de flux sanguin (30), et la saturation en oxygène acquise par lesdits moyens d'acquisition d'informations de saturation en oxygène, à ladite relation mémorisée dans ladite unité de mémorisation.

7. Dispositif selon la revendication 6, dans lequel lesdits moyens d'acquisition d'informations de saturation en oxygène ont
une source de lumière (33, 34) pour émettre une lumière ayant au moins deux longueurs d'onde différentes, un détecteur (35) pour détecter une lumière provenant de ladite source de lumière (33, 34) et diffusée par ladite surface corporelle, et
des moyens pour calculer une saturation en oxygène de l'hémoglobine sur la base des résultats de détection dudit détecteur (35).

8. Dispositif selon la revendication 6, dans lequel lesdits moyens d'acquisition d'informations de saturation en oxygène ont une unité opérationnelle pour entrer la saturation en oxygène sous forme de données numériques.

9. Dispositif selon la revendication 1, dans lequel ladite unité de détection thermométrique de première position (12) a une unité de tampon (13) pour placer un doigt, et
un capteur de température (20) pour mesurer la température dérivée de ladite première position du doigt placé sur ladite unité de tampon (13), ledit capteur de température (20) mesurant de préférence une température de rayonnement dérivée de ladite première position.

10. Dispositif de mesure de taux métabolique, comportant :

une unité de capteur de température environnementale (23) pour mesurer la température environnementale,

une unité de contact de surface corporelle (13) pour une mise en contact avec une première position de la surface corporelle,

un élément cylindrique, mis en contact avec ladite unité de contact de surface corporelle (13) et ayant une ouverture à une première extrémité,

un capteur thermométrique de rayonnement (27) monté à proximité de l'autre extrémité dudit élément cylindrique pour mesurer une chaleur rayonnante provenant de ladite première position,

des moyens d'acquisition d'informations de flux sanguin (30) pour acquérir des informations concernant le flux sanguin à ladite première position,

une source de lumière (33, 34) pour émettre une lumière ayant au moins deux longueurs d'onde différentes dans la première extrémité dudit élément de cylindre,

une unité de capteur de lumière (35) pour détecter une lumière qui interagit avec ladite surface corporelle,

des moyens d'acquisition de seconde température (1) pour acquérir une température d'une seconde position, la seconde position étant différente de ladite première position sur ladite surface corporelle,

une unité de traitement (45) pour calculer le taux métabolique en appliquant la température environnementale mesurée par ladite unité de capteur thermométrique environnementale (23), la température d'une première position mesurée par ladite unité de capteur thermométrique de rayonnement (27), la température de la seconde position acquise par lesdits moyens d'acquisition de température de seconde position (1), les informations de débit sanguin acquises par lesdits moyens d'acquisition d'informations de flux sanguin (30), et un résultat de détection de lumière détectée par ladite unité de détection de lumière, à une équation mémorisée au préalable du taux métabolique, et

une unité d'affichage (11) pour afficher la sortie de ladite unité de traitement (45).

**11.** Dispositif selon la revendication 10, dans lequel lesdits seconds moyens d'acquisition de température de position (1) sont

un thermomètre corporel, ou

un tampon opérationnel pour entrer la température corporelle sous forme de données numériques.

**12.** Dispositif selon la revendication 10, dans lequel

ladite première position doit être une partie d'un des quatre membres du sujet à mesurer, et

ledit capteur thermométrique de rayonnement (27) mesure la chaleur rayonnante dérivée de ladite partie.

**13.** Dispositif selon la revendication 10, comportant de plus une unité de mémorisation de données pour mémoriser une sortie de ladite unité de traitement (45), dans lequel des données mémorisées dans ladite unité de mémorisation de données sont affichées sur ladite unité d'affichage (11) d'une manière en série temporelle.

**14.** Dispositif selon la revendication 10, dans lequel ledit taux métabolique est

le taux métabolique de glucose, ou

le taux métabolique de graisse neutre et/ou cholestérol.

EP 1 774 903 B1

## FIG. 1

finger surface temperature

room temperature

amount of radiated heat

radiation temperature $T_{FS}$

room temperature $T_R$

Amount of received light I

blood flow rate $\omega_b$

body temperature $T_c$

arterial blood temperature

body temperature

amount of reserved heat

metabolic rate

haemoglobin absorbance $A_1$

haemoglobin absorbance $A_2$

tissue oxygen saturation

metabolic rate corresponding to blood glucose concentration.

FIG. 2

## FIG. 3

## FIG. 4

# FIG. 5

```
                    ┌─────────┐
                    │  start  │
                    └────┬────┘
                         ▼
        ┌─────────────────────────────┐
        │    circuit self diagnosis    │
        └──────────────┬──────────────┘
                       ▼
        ┌─────────────────────────────┐
        │         warming up           │
        └──────────────┬──────────────┘
                       ▼
        ┌─────────────────────────────┐
        │   please place your finger   │
        └──────────────┬──────────────┘
                       ▼
        ┌─────────────────────────────┐
        │         count down           │
        └──────────────┬──────────────┘
                       ▼
        ┌─────────────────────────────┐
        │     release your finger      │
        └──────────────┬──────────────┘
                       ▼
        ┌─────────────────────────────┐
        │  data processing in progress │
        └──────────────┬──────────────┘
                       ▼
        ┌─────────────────────────────┐
        │    result display: 142.5     │
        └──────────────┬──────────────┘
                       ▼
                  ┌─────────┐
                  │   end   │
                  └─────────┘
```

# FIG. 6 A

# FIG. 6 B

# FIG. 6 C

FIG. 7

830nm 780nm

temperature
measurement
data

conversion from
optical measurement
data to tissue oxygen
saturation and
blood flow data

conversion to
metabolic
rate

FIG. 8

push button switches

10a    10b    10c    10d

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1568311 A

- JP 2004329542 A

**Non-patent literature cited in the description**

- **PENNES H. H.** Analysis of tissue and arterial blood temperatures in the resting human forearm. *J. applied physiology,* 1948, vol. 1, 93-122